# EUROPÄISCHE PATENTANMELDUNG

(11) **EP 0 618 205 A1**
(43) Veröffentlichungstag der Anmeldung: **05.10.1994**
(21) Anmeldenummer: 94810182.9
(22) Anmeldetag: 23.03.1994
(51) Int. Cl.: C07D 401/14, C08K 5/3492, C07D 401/12

(54) **Wasserlösliche Triazinderivate zur photochemischen und thermischen Stabilisierung von Polyamidfasermaterialien**

(30) Priorität: 02.04.1993 CH 1021/93
(71) Anmelder: CIBA-GEIGY AG, CH-4002 Basel (CH)
(72) Erfinder: Reinehr, Dieter, Dr., D-79400 Kandern (DE); Bacher, Jean-Pierre, F-68220 Buschwiller (FR); Rembold, Manfred, Dr., CH-4147 Aesch (CH)

(57) **Zusammenfassung**

Wasserlösliche Triazinderivate der allgemeinen Formel 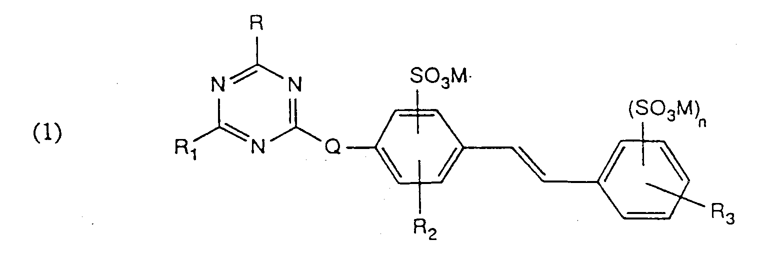
worin
R einen Rest der Formel 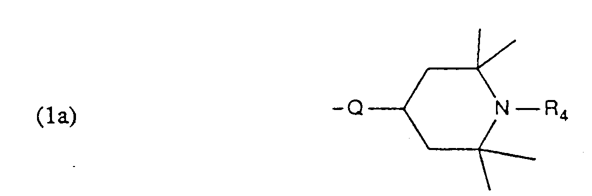
R₁ Halogen; Niederalkyl; Niederalkoxy; Niederalkylthio; Mononiederalkylamino; Diniederalkylamino; Cycloalkoxy; Cycloalkylthio; Cycloalkylamino; sulfoniertes oder nichtsulfoniertes Phenyl, Phenoxy, Phenylamino, Phenylthio, Phenylniederalkoxy, Phenylniederalkylamino oder Phenylniederalkylthio; einen Rest der Formel 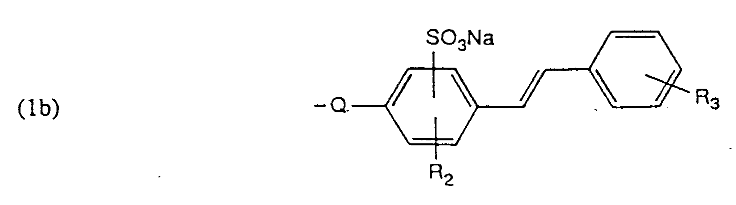
oder einen Rest der Formel (1a),
R₂ und R₃ unabhängig voneinander Wasserstoff, Halogen, Niederalkyl, Niederalkoxy oder einen Rest der Formel 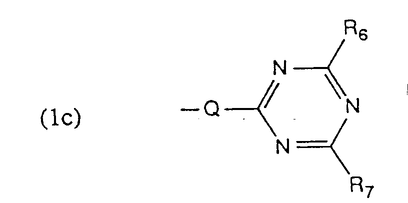
R₄ Wasserstoff, Oxyl, Hydroxy, Niederalkyl, Niederalkenyl, Niederalkoxy, Acyl oder Benzyl
R₅ Wasserstoff oder Niederalkyl,
R₆ und R₇ unabhängig voneinander Niederalkyl; Niederalkoxy; Niederalkylthio; Mononiederalkylamino; Diniederalkylamino; sulfoniertes oder nichtsulfoniertes Phenyl, Phenoxy, Phenylamino, Phenylthio, Phenylniederalkoxy, Phenylniederalkylamino oder Phenylniederalkylthio; einen Rest der Formel (1a);
M Wasserstoff, Alkalimetall, Erdalkalimetall, Ammonium oder einen organischen Ammoniumrest,
Q-O- oder -(NR₅)- und
n 0 oder 1 bedeuten.

Die Verbindungen stellen Vertreter der Klasse der sterisch gehinderten Amine ("HALS"-Stabilisatoren) dar. Sie eignen sich dazu, die thermische und photochemische Stabilität von ungefärbten und gefärbten Polyamid-Fasermaterialien zu erhöhen.

## Beschreibung

Die vorliegende Erfindung betrifft wasserlösliche Triazinderivate, Verfahren zur Herstellung dieser Verbindungen, ein Verfahren zur photochemischen und thermischen Stabilisierung von Polyamid-Fasermaterialien sowie das mit den erfindungsgemässen Verbindungen behandelte Fasermaterial.

Die wasserlöslichen Triazinderivate entsprechen der allgemeinen Formel 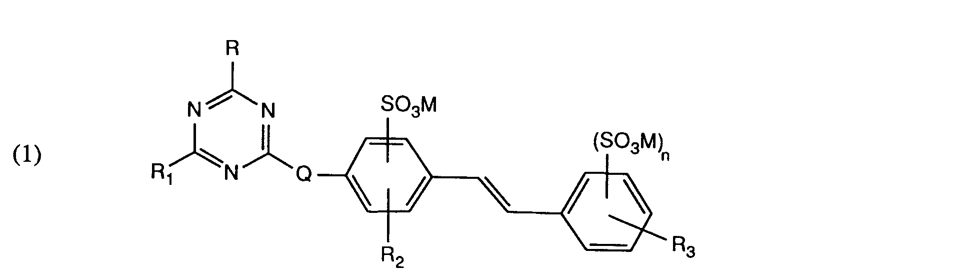
worin
R einen Rest der Formel 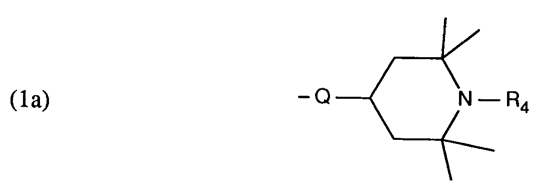
R₁ Halogen; Niederalkyl; Niederalkoxy; Niederalkylthio; Mononiederalkylamino; Diniederalkylamino; Cycloalkoxy; Cycloalkylthio; Cycloalkylamino; sulfoniertes oder nichtsulfoniertes Phenyl, Phenoxy, Phenylamino, Phenylthio, Phenylniederalkoxy, Phenylniederalkylamino oder Phenylniederalkylthio; einen Rest der Formel 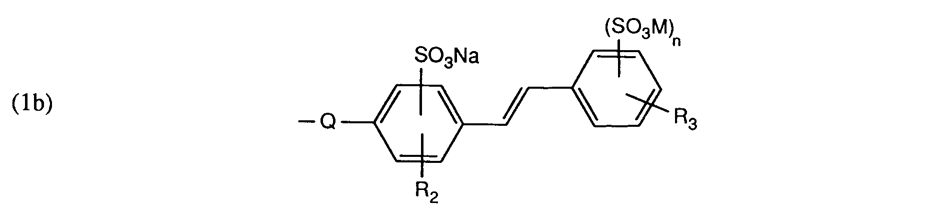
oder einen Rest der Formel (1a),
R₂ und R₃ unabhängig voneinander Wasserstoff, Halogen, Niederalkyl, Niederalkoxy oder einen Rest der Formel 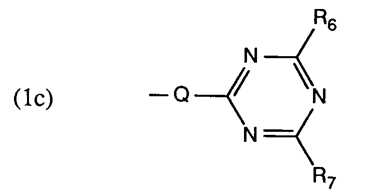
R₄ Wasserstoff, Oxyl, Hydroxy, Niederalkyl, Niederalkenyl, Niederalkoxy, Acyl oder Benzyl
R₅ Wasserstoff oder Niederalkyl,
R₆ und R₇ unabhängig voneinander Niederalkyl; Niederalkoxy; Niederalkylthio; Mononiederalkylamino; Diniederalkylamino; sulfoniertes oder nichtsulfoniertes Phenyl, Phenoxy, Phenylamino, Phenylthio, Phenylniederalkoxy, Phenylniederalkylamino oder Phenylniederalkylthio; oder einen Rest der Formel (1a);
M Wasserstoff, Alkalimetall, Erdalkalimetall, Ammonium oder einen organischen Ammoniumrest,
Q -O- oder-(NR₅)- und
n 0 oder 1 bedeuten.

Bei der Definition der Reste R₁ bis R₇ stellen Niederalkyl, Niederalkoxy, Niederalkylthio, Mononiederalkylamino und Diniederalkylamino solche Gruppen oder Gruppenbestandteile dar, die 1 bis 5, insbesondere 1 bis 3 Kohlenstoffatome aufweisen. Beispiele für derartige Gruppen sind Methyl, Ethyl, n-Propyl, Isopropyl, n-Butyl, sek.Butyl, tert. Butyl, Amyl oder Isoamyl bzw. Methoxy, Ethoxy, Isopropoxy, Isobutoxy, tert.-Butoxy oder tert.-Amyloxy bzw. Methylthio, Ethylthio, Propylthio oder Butylthio.

Niederalkenyl bedeutet beispielsweise Vinyl, Propenyl, Butenyl oder vorzugsweise Allyl.

Phenylniederalkylamino bedeutet beispielsweise Phenethyl-, Phenylpropyl-, Phenylbutyl- oder vorzugsweise Benzylamino.

Halogen bei den Resten R und R₂ bedeutet Fluor, Brom oder vorzugsweise Chlor.

Bei der Definition des Restes R₄ bedeutet Acyl besonders Formyl oder Niederalkanoyl, wie z.B. Acetyl oder Propionyl, oder Benzoyl.

Als Beispiele für Alkalimetalle seien Lithium, Natrium oder Kalium genannt. Bevorzugt ist Natrium. Beispiele für Erdalkalimetalle sind Calcium und Magnesium.

Als organischer Ammoniumrest kommt Trimethylammonium oder vorzugsweise Triethylammonium in Betracht.

Niederalkylamino, Diniederalkylamino und Cycloniederalkylamino können durch Halogen, Niederalkoxy, Hydroxy, Carboxy oder Carboxyniederalkyl substituiert sein. Nieder-alkoxy und Cycloalkoxy können durch Niederalkoxy substituiert sein. Niederalkylthio und Cycloalkylthio können durch Alkoxy oder Hydroxy substituiert sein. Phenyl kann durch Niederalkyl, Niederalkoxy oder Halogen substituiert sein.

Von praktisch wichtiger Bedeutung sind wasserlösliche Triazinderivate, bei denen in Formel (1) R₁ Halogen; Niederalkoxy; Niederalkylthio; sulfoniertes oder nichtsulfoniertes Phenoxy, Phenylamino, Phenylthio; oder einen Rest der Formel (1a) bedeutet.

Von besonderem Interesse sind dabei Triazinderivate, bei denen R₁ einen Rest der Formel (1a) und solche Triazinderivate, bei denen Q -(NR₅)- bedeutet.

Weitere wichtige Triazinderivate der Formel (1) sind diejenigen Verbindungen, bei denen R₃ Wasserstoff oder Niederalkyl bedeutet.

Ferner stehen Triazinderivate der Formel (1) im Vordergrund, bei denen
R₃ einen Rest der Formel (lc) bedeutet und ganz besonders diejenigen Verbindungen, bei denen in Formel (1c)
R₆ Wasserstoff oder Niederalkyl bedeutet.

Besonders interessant sind Triazinderivate der Formel (1), bei denen
R und R₁ einen Rest der Formel (1a),
R₄ Wasserstoff oder Niederalkyl und
n = 0 bedeuten.

Die Herstellung der wasserlöslichen Triazinderivate entsprechend Formel (1) kann auf verschiedene Art und Weise erfolgen. Ausgangsverbindung ist im allgemeinen eine 2,4,6-Trihalogen-s-triazinverbindung, wobei Cyanurchlorid bevorzugt ist.

Die Herstellung der erfindungsgemässen Triazinderivate der Formel (1) erfolgt z.B. dadurch, dass man 1 Mol einer 2,4,6-Trihalogen-s-triazinverbindung nacheinander mit einem oder 2 Mol einer Stilbenverbindung der Formel 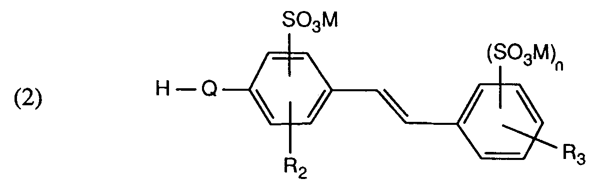
und 1 bzw. 2Mol der Piperidinverbindung der Formel 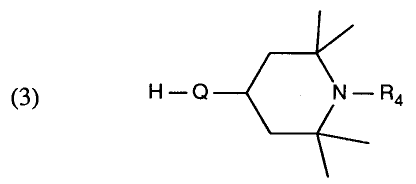
umsetzt. R₂ und R₃ bedeuten dabei, unabhängig voneinander, Wasserstoff, Niederalkyl und Niederalkoxy. R₄, M, Q und n haben die in den Formeln (1a) und (1b) angegebenen Bedeutungen.

Bedeutet R₃ in Formel (1b) einen Rest der Formel (1c), erfolgt die Herstellung der erfindungsgemässen Triazinderivate der Formel (1) so, dass man nacheinander 1 Mol der Stilbenverbindung der Formel (2) mit 2 Mol einer 2,4,6-Trihalogen-s-triazinverbindung und anschliessend mit 4 Mol einer Piperidinverbindung der Formel (3) oder mit 2 Mol einer Piperidinverbindung der Formel (3) und 2 Mol einer entsprechenden Mononiederalkylamino-, Diniederalkylamino-, Phenylniederalkylamino-, Phenoxy-, Phenylamino-, Niederalkoxy- oder Niederalkylthio-Verbindung umsetzt.

Die bei den Kondensationsreaktionen entstehende Halogenwasserstoffsäure kann durch das Endprodukt selbst oder durch Hinzufügen einer weiteren Base, wie beispielsweise wässrigem Ammoniak, Alkalimetallhydroxiden, Alkalimetallcarbonaten, -hydrogen-carbonaten oder einer organischen Base, wie beispielsweise Triethylamin, abgefangen werden. Vorzugsweise wird als Base ein Alkalimetallcarbonat, wie z.B. Natriumcarbonat, verwendet.

Die Umsetzungen erfolgen zweckmässigerweise in wässriger Lösung gegebenenfalls mit Zusatz eines aprotischen organischen Lösungsmittels, wie beispielsweise Aceton oder Methylethylketon. Die als Ausgangsverbindungen verwendeten 2,4,6-Trihalogen-s-triazinverbindungen sind allgemein bekannt. Sie werden dabei vorzugsweise als wässrige Suspensionen eingesetzt. Besonders bevorzugte Ausgangsverbindung ist Cyanurchlorid.

Alle Verbindungen gemäss Formel (1) werden vorzugsweise als Natriumsalze eingesetzt. Dazu werden sie beispielsweise mit der äquivalenten Menge Natronlauge gelöst und als Lösung, Dispersion oder Emulsion für eine Anwendung formuliert.

Die neuartigen Verbindungen stellen Vertreter der Klasse der sterisch gehinderten Amine ("HALS"-Stabilisatoren) dar. Sie sind geeignet, die thermische und photochemische Stabilität von ungefärbten und gefärbten Polyamid-Fasermaterialien zu erhöhen. Das Verfahren zur photochemischen Stabilisierung von Polyamid-Fasermaterialien mit Hilfe dieser Verbindungen bildet daher einen weiteren Gegenstand der vorliegenden Erfindung. Das Verfahren ist dadurch gekennzeichnet, dass man das gefärbte oder ungefärbte Polyamid-Fasermaterial mit wasserlöslichen Triazinderivaten der allgemeinen Formel 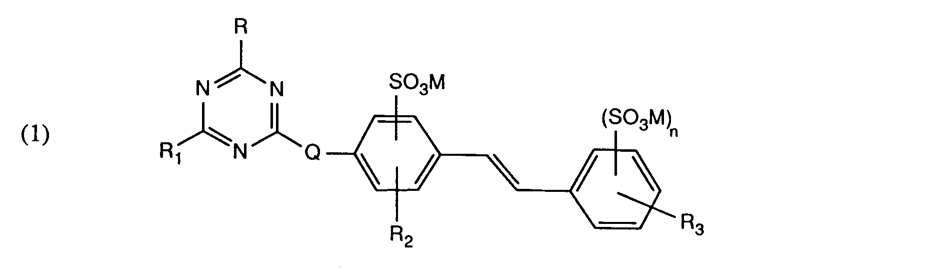
behandelt, worin
R einen Rest der Formel 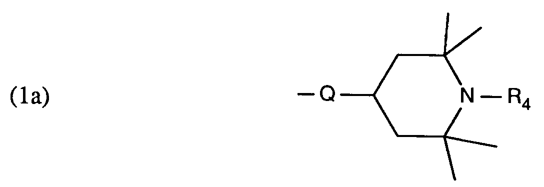
R₁ Halogen; Niederalkyl; Niederalkoxy; Niederalkylthio; Mononiederalkylamino; Diniederalkylamino; Cycloalkoxy; Cycloalkylthio; Cycloalkylamino; sulfoniertes oder nichtsulfoniertes Phenyl, Phenoxy, Phenylamino, Phenylthio, Phenylniederalkoxy, Phenylniederalkylamino oder Phenylniederalkylthio; einen Rest der Formel 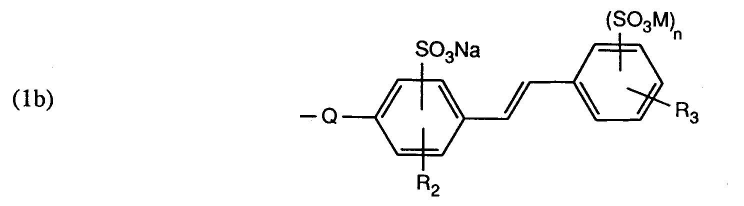
oder einen Rest der Formel (1a),
R₂ und R₃ unabhängig voneinander Wasserstoff, Halogen, Niederalkyl, Niederalkoxy oder einen Rest der Formel 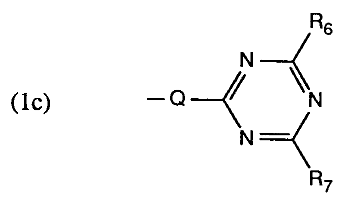
R₄ Wasserstoff, Oxyl, Hydroxy, Niederalkyl, Niederalkenyl, Niederalkoxy, Acyl oder Benzyl
R₅ Wasserstoff oder Niederalkyl,
R₆ und R₇ unabhängig voneinander Niederalkyl; Niederalkoxy; Niederalkylthio; Mononiederalkylamino; Diniederalkylamino; sulfoniertes oder nichtsulfoniertes Phenyl, Phenoxy, Phenylamino, Phenylthio, Phenylniederalkoxy, Phenylniederalkylamino oder Phenylniederalkylthio;
M Wasserstoff, Alkalimetall, Erdalkalimetall, Ammonium oder einen organischen Ammoniumrest,
Q -O- oder -(NR₅)- und
n 0 oder 1 bedeuten.

Die Verbindungen der Formel (1 ) können aus üblichen Flotten nach gängigen Methoden auf die Polyamid-Fasermaterialien aufgebracht werden. Erfindungsgemäss werden sie aus wässrigem Bad appliziert, das die Verbindungen in einer Menge von 0,005 bis 10 Gew.-%, vorzugsweise 0,05 bis 2 Gew.-% enthält. Vorzugsweise wird die Behandlung mit den erfindungsgemässen Verbindungen und die Färbung im gleichen Applikationsbad vorgenommen. Man kann dabei so vorgehen, dass dem wässrigen Applikationsbad zuerst der UV-Absorber zugefügt, das entsprechende Fasermaterial behandelt und anschliessend die Färbung durchgeführt wird, oder man kann gleichzeitig dem Bad den UV-Absorber und den Farbstoff zugeben oder zunächst die Färbung durchführen und dann die Behandlung mit dem UV-Absorber vornehmen. Die gleichzeitige Applikation von UV-Absorber und Farbstoffen ist bevorzugt. Die Applikation kann dabei nach einem Auszieh- oder Kontinueverfahren erfolgen.

Beim Ausziehverfahren kann das Flottenverhältnis innerhalb eines weiten Bereiches gewählt werden, z.B. 1:5 bis 1:300, vorzugsweise 1:10 bis 1:50. Man arbeitet zweckmäßig bei einer Temperatur von 30 bis 120°C, vorzugsweise 50 bis 98°C.

Beim Kontinue-Verfahren beträgt der Flottenauftrag zweckmäßig 30-400 Gew.-%, vorzugsweise 75-250 Gew.-%. Zur Fixierung der applizierten Farbstoffe und der bekannten und neuen Verbindungen wird das Fasermaterial einer Hitzebehandlung unterworfen. Der Fixierprozess kann auch nach der Kaltverweilmethode erfolgen.

Die Hitzebehandlung erfolgt vorzugsweise durch ein Dämpfverfahren unter Behandlung in einem Dämpfer mit gegebenenfalls überhitztem Dampf bei einer Temperatur von 98 bis 105°C während z.B. 1 bis 7, vorzugsweise 1 bis 5 Minuten. Die Fixierung der Farbstoffe und der Verbindungen der Formel (1) gemäss dem Kaltverweilverfahren kann durch Lagerung der imprägnierten und vorzugsweise aufgerollten Ware bei Raumtemperatur (15 bis 30°C), z.B. während 3 bis 24 Stunden erfolgen, wobei die Kaltverweilzeit bekanntlich von der Art des applizierten Farbstoffes abhängig ist.

Nach Beendigung des Färbeprozesses bzw. der Fixierung werden die fertig hergestellten Fasermaterialien auf übliche Weise gespült und getrocknet.

Mit dem erfindungsgemässen Verfahren erhalten sowohl Polyamid-Färbungen als auch Polyamid-Fasern eine gute thermische und photochemische Stabilität.

Als die erfindungsgemäss zu stabilisierenden Färbungen kommen solche in Betracht, die durch Säure- oder Metallkomplexfarbstoffe, z.B. 1:2-Chrom, 1:2-Kobaltkomplexfarbstoffe oder Kupferkomplexfarbstoffe oder Dispersions- und Reaktivfarbstoffe erzeugt werden.

Beispiele für solche Farbstoffe sind in Colour Index, 3. Auflage, 1971, Band 4, beschrieben.

Unter Polyamidfasermaterial wird solches aus synthetischem Polyamid, wie z.B. Polyamid 6, Polyamid 6,6 oder Polyamid 12, sowie modifiziertes Polyamid, z.B. basisch anfärbbares Polyamid verstanden. Neben den reinen Polyamidfasern kommen vor allem auch Fasermischungen aus Polyurethan und Polyamid in Betracht, so z.B. Trikotmaterial aus Polyamid/Polyurethan im Mischungsverhältnis 70:30. Grundsätzlich kann das reine oder gemischte Polyamidfasermaterial in den verschiedensten Verarbeitungsformen vorliegen, wie z.B. als Faser, Garn, Gewebe, Gewirke, Vlies oder Flormaterial.

Das vorliegende Verfahren eignet sich besonders vorteilhaft zur Behandlung von Polyamidfasermaterial, das Licht und Hitze ausgesetzt wird und z.B. als Autopolsterstoff oder Teppich Verwendung findet.

Die folgenden Beispiele veranschaulichen die Erfindung. Teile bedeuten Gewichtsteile und Prozente Gewichtsprozente.

### Herstellunwsbeispiele der neuen Verbindungen

### Beispiel 1:

17 g 4,4′-Diaminostilben-2,2′-disulfonsäure werden in 100 ml Wasser aufgelöst und mit 1n Natronlauge auf einen pH-Wert von 7,5 eingestellt. Diese Lösung wird zu einem Gemisch von 18,4 g Cyanurchlorid, 200 ml Aceton und 10 g Eis bei 0-5°C zugetropft. Der pH-Wert wird dabei auf 2,5 bis 3 gehalten. Nach einer Rührzeit von 2 Stunden bei 5°C wird eine Lösung von 17,6 g Metanilsäure zugetropft, der pH-Wert auf 6,5 bis 7 erhöht und während 2 Stunden bei 20 bis 25°C nachgerührt. Nach der anschliessenden Zugabe von 31,2 g 4-Amino-2,2,6,6-tetramethylpiperidin wird die Temperatur auf 50°C, der pH-Wert auf 10-11 erhöht und die Reaktionsmischung 2 Stunden bei 50°C gerührt. Die hellbraune Lösung wird dann eingedampft und das erhaltene Rohprodukt zweimal aus Wasser umkristallisiert. Man erhält 56 g ( 88% d.Th.) der hellbeigen Verbindung der Formel 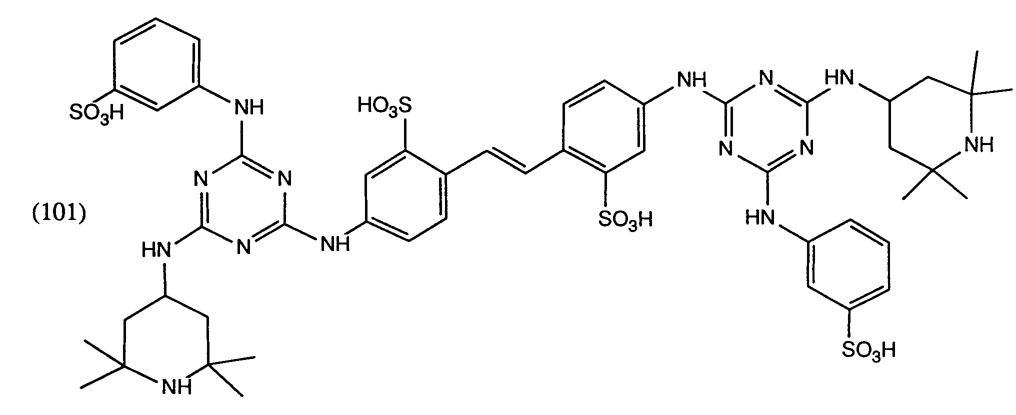
Absoptionsmaximum: λₘₐₓ 354 nm (gemessen in Acetonitril)

### Beispiele 2 und 3:

Nach der in Beispiel 1 beschriebenen Methode werden folgende Verbindungen der allgemeinen Formel (A) hergestellt:

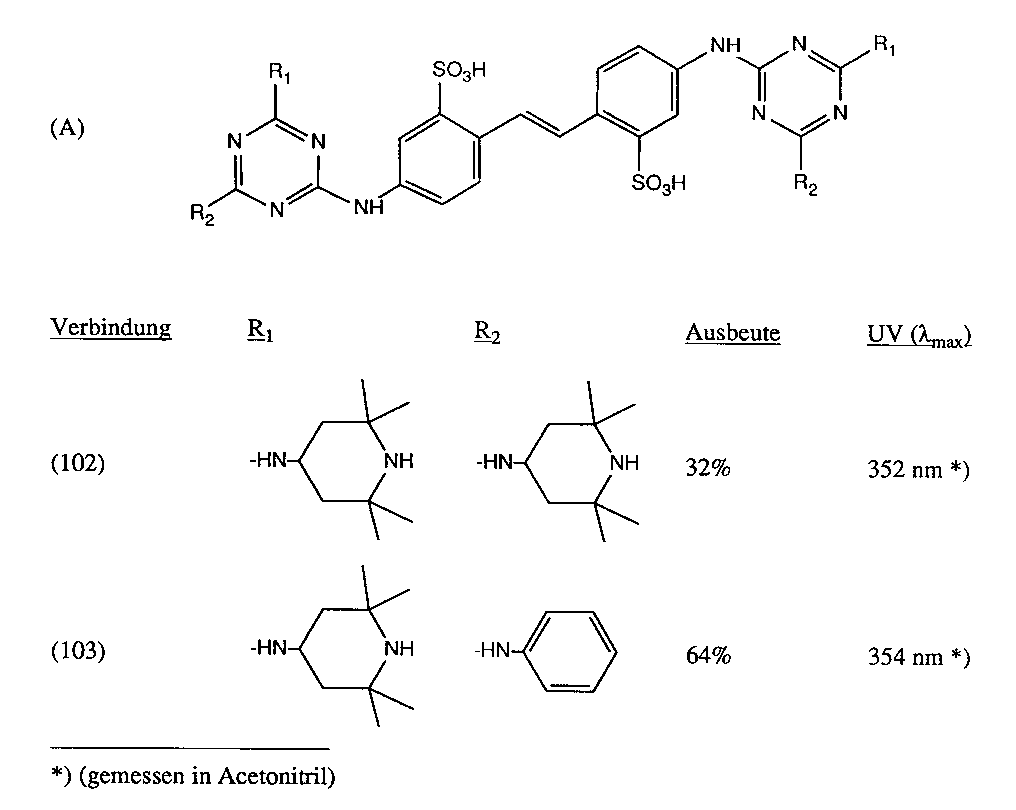

Beispiele 4 bis 6: Die Verbindungen der allgemeinen Formel (B) werden nach der in Beispiel 1 beschriebenen Methode hergestellt:

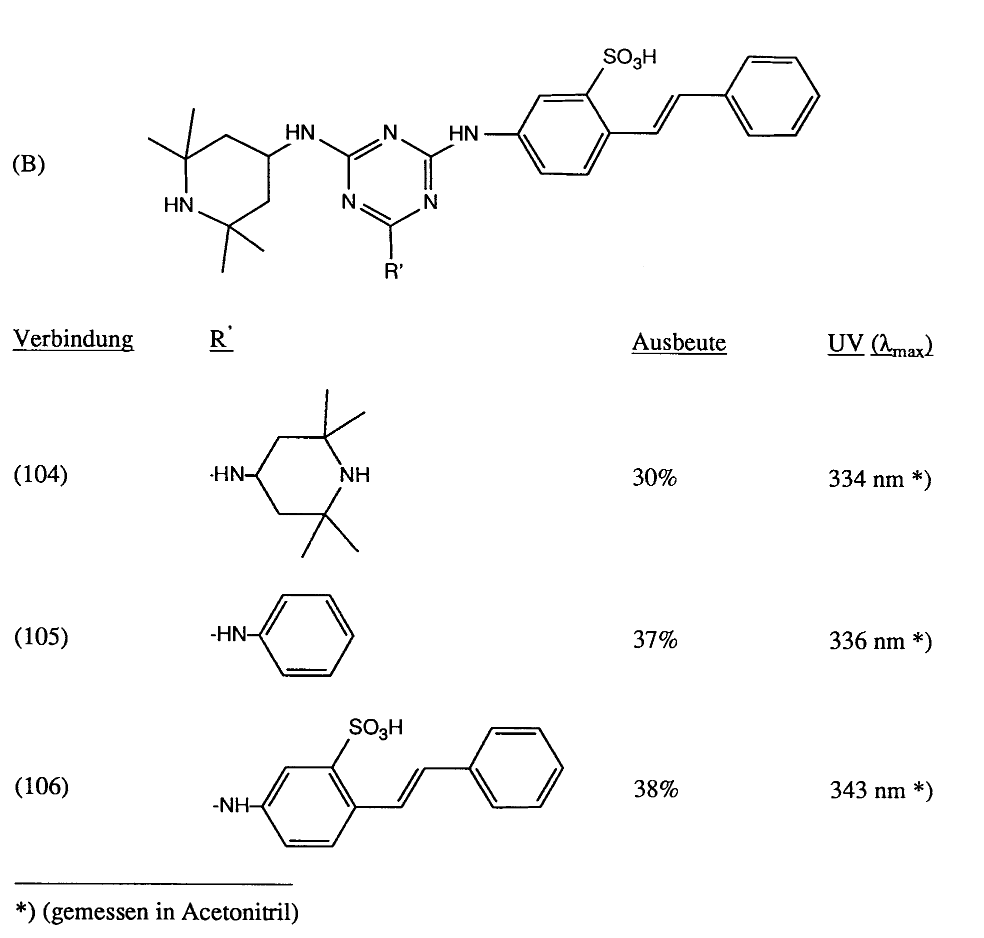

### Anwendungsbeispiele:

Beispiel 7: 3 Muster von je 10 g einer PA 6 Maschenware (Lilion Texturtricot 5-4003) werden in einem Färbeapparat, z.B. Vistacolor der Firma Zeller bei einem Flottenverhältnis von 1:25 gefärbt. Die 3 Bäder enthalten noch folgende Zusätze:
1 g/l Na-Phosphat im Verhältnis 25 Teile Mono-Na zu 175 Teilen Di-Na Salz (pH 7,5), 1%, bezogen auf das textile Material, eines nichtionischen Egalisierhilfsmittels
Während die Flotte 1 keine weiteren Zusätze enthält, werden den Flotten 2 und 3 jeweils noch 1%, bezogen auf das textile Material, von den Verbindungen der Formel (104) und (105) zugegeben.

In die so vorbereiteten Flotten geht man bei 45°C ein und erhitzt innerhalb von 45 Minuten auf 95°C. Nach einer Färbezeit von 40 Minuten bei 95°C kühlt man auf 60°C ab, spült mit kaltem Wasser, zentrifugiert und trocknet das Gewebe bei Raumtemperatur.

Die so behandelten Muster werden entsprechend DIN 75.202 (FAKRA) belichtet und ihre Reissfestigkeiten nach SN 198.461 gemessen. Die Ergebnisse sind in Tabelle 1 dargestellt:

**Tabelle 1:**

| Zusatz zum Färbebad | Reissfestigkeit nach 120 h FAKRA Belichtung in % |
|---|---|
| kein Zusatz | 10 |
| 1% der Verbindung (104) | 71 |
| 1% der Verbindung (105) | 63 |
| 1% der Verbindung (106) | 56 |

3 weitere, entsprechend Beispiel 1 behandelte Muster, werden einem Hitzetest unterzogen. Die Muster werden dabei für 72 Stunden in einem Umluftofen bei 140°C gealtert und anschliessend der Reisswert nach SN 198.461 bestimmt. Die Ergebnisse sind in Tabelle 2 dargestellt:

**Tabelle 2:**

| Zusatz zum Färbebad | Reissfestigkeit nach 72 h bei 140°C in % |
|---|---|
| kein Zusatz | 16 |
| 1% der Verbindung (104) Ausziehgrad 94% | 43 |
| 1% der Verbindung (105) Ausziehgrad 90% | 25 |
| 1% der Verbindung (106) Ausziehgrad 71% | 23 |

Die mit den erfindungsgemässen Verbindungen behandelten Gewirke ergeben sowohl bei photochemischer als auch thermischer Beanspruchung deutlich höhere Reissfestigkeiten.

## Patentansprüche

1. Wasserlösliche Triazinderivate der allgemeinen Formel 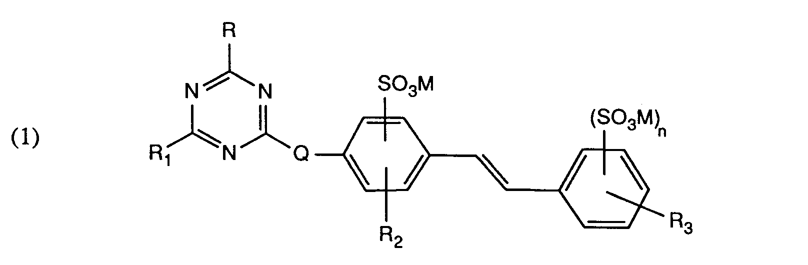 worin
R einen Rest der Formel 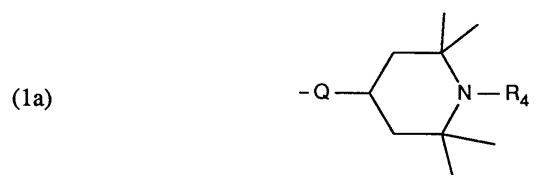 R₁ Halogen; Niederalkyl; Niederalkoxy; Niederalkylthio; Mononiederalkylamino; Diniederalkylamino; Cycloalkoxy; Cycloalkylthio; Cycloalkylamino; sulfoniertes oder nichtsulfoniertes Phenyl, Phenoxy, Phenylamino, Phenylthio, Phenylniederalkoxy, Phenylniederalkylamino oder Phenylniederalkylthio; einen Rest der Formel 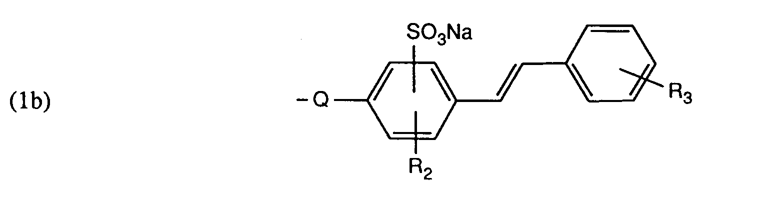 oder einen Rest der Formel (1a),
R₂ und R₃ unabhängig voneinander Wasserstoff, Halogen, Niederalkyl, Niederalkoxy oder einen Rest der Formel 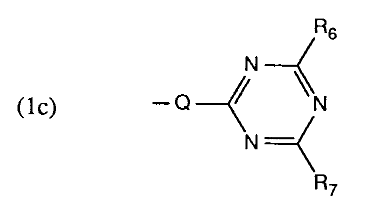 R₄ Wasserstoff, Oxyl, Hydroxy, Niederalkyl, Niederalkenyl, Niederalkoxy, Acyl oder Benzyl
R₅ Wasserstoff oder Niederalkyl,
R₆ und R₇ unabhängig voneinander Niederalkyl; Niederalkoxy; Niederalkylthio; Mononiederalkylamino; Diniederalkylamino; sulfoniertes oder nichtsulfoniertes Phenyl, Phenoxy, Phenylamino, Phenylthio, Phenylniederalkoxy, Phenylniederalkylamino oder Phenylniederalkylthio; einen Rest der Formel (1a);
M Wasserstoff, Alkalimetall, Erdalkalimetall, Ammonium oder einen organischen Ammoniumrest,
Q -O- oder -(NR₅)- und
n 0 oder 1 bedeuten.

2. Triazinderivate nach Anspruch 1, dadurch gekennzeichnet, dass R₁ Halogen; Niederalkoxy; Niederalkylthio: sulfoniertes oder nichtsulfoniertes Phenoxy, Phenylamino, Phenylthio; oder einen Rest der Formel (1a) bedeutet.

3. Triazinderivate nach Anspruch 1 oder 2, dadurch gekennzeichnet, dass R₁ einen Rest der Formel (1a) bedeutet.

4. Triazinderivate nach einem der Ansprüche 1 bis 3, dadurch gekennzeichnet, dass Q -(NR₅)- bedeutet.

5. Triazinderivate nach einem der Ansprüche 1 bis 4, dadurch gekennzeichet, dass R₃ Wasserstoff oder Niederalkyl bedeutet.

6. Triazinderivate nach einem der Ansprüche 1 bis 4, dadurch gekennzeichnet, dass R₃ einen Rest der Formel (1c) bedeutet.

7. Triazinderivate nach Anspruch 6, dadurch gekennzeichnet, dass in Formel (1c) R₆ Wasserstoff oder Niederalkyl bedeutet.

8. Triazinderivate nach Anspruch 1, dadurch gekennzeichnet, dass
R und R₁ einen Rest der Formel (1a),
R₄ Wasserstoff oder Niederalkyl und
n = 0 bedeuten.

9. Verfahren zur Herstellung der Triazinverbindungen der Formel (1), dadurch gekennzeichnet dass man dass man 1 Mol einer 2,4,6-Trihalogen-s-triazinverbindung nacheinander mit einem oder 2 Mol einer Stilbenverbindung der Formel 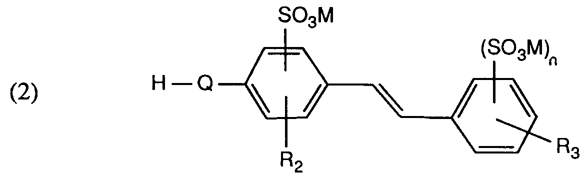 und 1 bzw. 2 Mol der Piperidinverbindung der Formel 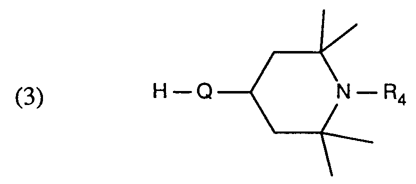 umsetzt, wobei
R₂ und R₃, unabhängig voneinander, Wasserstoff, Niederalkyl oder Niederalkoxy und
R₄ Wasserstoff, Oxyl, Hydroxy, Niederalkyl, Niederalkenyl, Niederalkoxy, Acyl oder Benzyl
Q -O- oder -(NR₅)-,
M Wasserstoff, Alkalimetall, Erdalkalimetall, Ammonium oder einen organischen Ammoniumrest, und
n 0 oder 1 bedeuten.

10. Verfahren zur Herstellung der Triazinverbindungen der Formel (1), worin R₃ in Formel (1b) einen Rest der Formel (1c) bedeutet, dadurch gekennzeichnet, dass man nacheinander 1 Mol der Stilbenverbindung der Formel (2) mit 2 Mol einer 2,4,6-Trihalogen-s-triazinverbindung und anschliessend mit 4 Mol einer Piperidinverbindung der Formel (3) oder mit 2 Mol einer Piperidinverbindung der Formel (3) und 2 Mol einer entsprechenden Mononiederalkylamino-, Diniederalkylamino-, Phenylniederalkylamino-, Phenoxy-, Phenylamino-, Niederalkoxy- oder Niederalkylthio-Verbindung umsetzt.

11. Verfahren zur photochemischen und thermischen Stabilisierung von Polyamidfasermaterialien, dadurch gekennzeichnet, dass man gefärbte oder ungefärbte Polyamid-Fasermaterialien mit wasserlöslichen Triazinderivaten der allgemeinen Formel 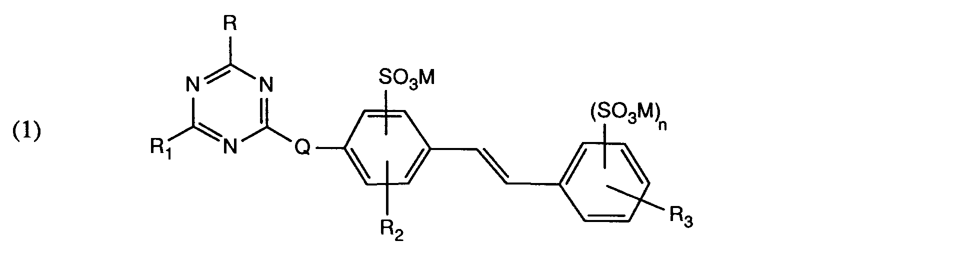 behandelt, worin
R einen Rest der Formel 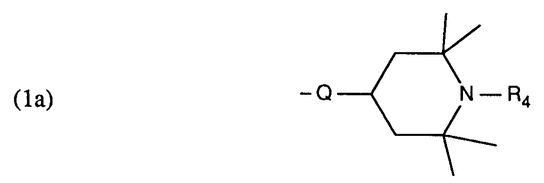 R₁ Halogen; Niederalkyl; Niederalkoxy; Niederalkylthio; Mononiederalkylamino; Diniederalkylamino; Cycloalkoxy; Cycloalkylthio; Cycloalkylamino; sulfoniertes oder nichtsulfoniertes Phenyl, Phenoxy, Phenylamino, Phenylthio, Phenylniederalkoxy, Phenylniederalkylamino oder Phenylniederalkylthio; einen Rest der Formel 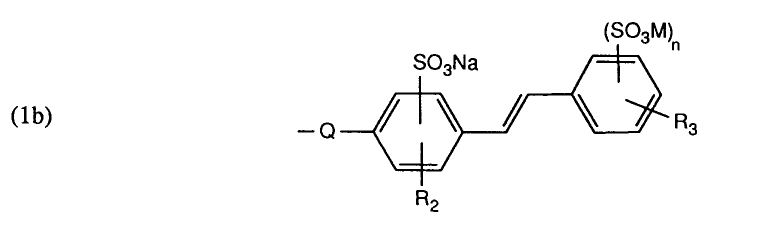 oder einen Rest der Formel (1a),
R₂ und R₃ unabhängig voneinander Wasserstoff, Halogen, Niederalkyl, Niederalkoxy oder einen Rest der Formel 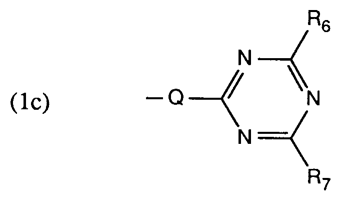 R₄ Wasserstoff, Oxyl, Hydroxy, Niederalkyl, Niederalkenyl, Niederalkoxy, Acyl oder Benzyl
R₅ Wasserstoff oder Niederalkyl,
R₆ und R₇ unabhängig voneinander Niederalkyl; Niederalkoxy; Niederalkylthio; Mononiederalkylamino; Diniederalkylamino; sulfoniertes oder nichtsulfoniertes Phenyl, Phenoxy, Phenylamino, Phenylthio, Phenylniederalkoxy, Phenylniederalkylamino oder Phenylniederalkylthio; einen Rest der Formel (1a);
M Wasserstoff, Alkalimetall, Erdalkalimetall, Ammonium oder einen organischen Ammoniumrest,
Q -O- oder -(NR₅)- und
n 0 oder 1 bedeuten.

12. Das gemäss dem Verfahren nach Anspruch 11 behandelte Fasermaterial.
